# EUROPÄISCHE PATENTSCHRIFT

(11) **EP 2 967 997 B1**
(45) Veröffentlichungstag und Bekanntmachung des Hinweises auf die Patenterteilung: **09.09.2020**
(21) Anmeldenummer: 14703324.5
(22) Anmeldetag: 04.02.2014
(51) Int. Cl.: A61F 9/008

(54) **ERZEUGUNG GEKRÜMMTER SCHNITTE IM INNEREN DER AUGENHORNHAUT**
CREATION OF CURVED CUTS IN THE INSIDE OF THE EYE CORNEA
RÉALISATION D'ENTAILLES COURBÉES À L'INTÉRIEUR DE LA CORNÉE

(30) Priorität: 14.03.2013 DE 102013204496
(43) Veröffentlichungstag der Anmeldung: 20.01.2016
(73) Patentinhaber: Carl Zeiss Meditec AG, 07745 Jena (DE)
(72) Erfinder: BISCHOFF, Mark, 07749 Jena (DE); STOBRAWA, Gregor, 07743 Jena (DE)
(74) Vertreter: Patentanwälte Geyer, Fehners & Partner mbB
(86) Internationale Anmeldenummer: PCT/EP2014/052167
(87) Internationale Veröffentlichungsnummer: WO 2014/139732

(56) Entgegenhaltungen:
- DE-A1- 10 202 036
- DE-A1-102008 056 488
- DE-A1-102008 062 658
- US-A1- 2004 243 112
- US-A1- 2012 078 240
- KARSTEN KOENIG ET AL: "Intratissue surgery with 80 MHz nanojoule femtosecond laser pulses in the near infrared", OPTICS EXPRESS, Bd. 10, Nr. 3, 11. Februar 2002 (2002-02-11), Seiten 171-176, XP055108893, ISSN: 1094-4087, DOI: 10.1364/OE.10.000171

## Beschreibung

Die Erfindung bezieht sich auf eine Vorrichtung zum Isolieren eines Lentikels in der Hornhaut eines Auges, die aufweist eine Laserstrahlquelle, die ausgebildet ist, gepulste Laserstrahlung abzugeben mit einer in die Hornhaut eindringenden Wellenlänge, einer Strahlformungseinrichtung, die aufweist eine Strahloptik, welche die gepulste Laserstrahlung in die Hornhaut in einen Fokus bündelt, und einer Strahlablenkeinrichtung, die einen Fokus der Strahlung in der Hornhaut verschiebt, wobei eine Steuereinrichtung vorgesehen ist, die ausgebildet ist, die Laserstrahlquelle und die Strahlformungseinrichtung anzusteuern, um in der Hornhaut ein Lentikel zu isolieren, welches durch eine Schnittfläche begrenzt ist.

Die Erfindung bezieht sich weiter auf ein Verfahren zum Isolieren eines Lentikels in der Hornhaut eines Auges, wobei in der Hornhaut mindestens eine Schnittfläche festgelegt wird, die das Lentikel umgrenzt und die Schnittfläche in der Hornhaut durch die Abgabe gepulster Laserstrahlung erzeugt wird, wobei gepulste Laserstrahlung verwendet wird, die eine in die Hornhaut eindringende Wellenlänge hat und ein Fokus der Laserstrahlung in der Hornhaut verschoben wird, und wobei das Verfahren keine chirurgische Behandlung des lebenden menschlichen oder tierischen Auges vornimmt.

Für die Abbildungseigenschaften des Auges ist die Form der Vorderfläche der Augenhornhaut von Bedeutung. Es ist deshalb seit langem bekannt, für eine Fehlsichtigkeitskorrektur die Augenhornhaut zu verändern, mit dem Ziel die Vorderfläche der Augenhornhaut und damit deren Brechungseigenschaften zu modifizieren und so eine Fehlsichtigkeit auszugleichen. Im Stand der Technik wurden dazu Operationsverfahren entwickelt, welche an der Augenhornhaut eine Lamelle lösen, diese abklappen und dann aus dem derart freigelegten Inneren der Augenhornhaut Material abtragen. Anschließend wird die Lamelle wieder zurückgeklappt und die Augenhornhaut hat aufgrund des Materialabtrages eine anders geformte Vorderfläche. Dieses Korrekturprinzip ist im Stand der Technik u. a. mit der Bezeichnung LASIK abgekürzt und sei nachfolgend als lamellenlösende Fehlsichtigkeitskorrektur bezeichnet. Das Lösen der Lamelle erfolgte in frühen Ausführungsformen mittels eines mechanischen Keratoms. Die Augenhornhaut wird durch ein ebenes Kontaktglas flachgedrückt und mittels des mechanischen Keratoms ein die Lamelle erzeugender Schnitt ausgeführt. In einer Weiterbildung werden mittlerweile sogenannte Laserkeratome verwendet. Hierzu ist u. a. ein Laserkeratom der Ziemer Ophthalmie Systems AG, Port, Schweiz, bekannt. Es ist hinsichtlich seiner Strahlablenkung zur Erzeugung der abzuklappenden Lamelle ausgebildet. Ein weiteres Laserkeratom wurde von der Intralase Inc., USA, entwickelt, die mittlerweile der Abbott Laboratories, Illinois, USA, gehört. Beide Laserkeratome arbeiten mit gepulster Laserstrahlung, wobei sich Repetitionsraten und Pulsenergien unterscheiden.

Die US 2012/0078240 A1 offenbart ein Kombinationsgerät, das sowohl als Laserkeratom arbeitet, als auch im freigelegten Inneren der Augenhornhaut Material abträgt. Die DE 10202036 A1 und die DE 102008062658 A1 offenbaren Kurzpulslasersysteme zur Hornhautchirurgie.

Die chirurgische Fehlsichtigkeitskorrektur wurde weiterentwickelt zu Verfahren, die in der Hornhaut Material isolieren und extrahieren. Das Material hat üblicherweise die Form eines Lentikels, weshalb diese Verfahren hier lentikelextrahierende Verfahren bzw. Vorrichtungen genannt werden. Auch wird das zu isolierende und extrahierende Volumen der Anschaulichkeit halber mit der Bezeichnung "Lentikel" versehen, auch wenn in bestimmten Anwendungsfällen ein nicht lentikelförmiges Volumen zu isolieren und extrahieren ist.

Die eingangs genannten Vorrichtungen und Verfahren beziehen sich auf das Prinzip der Lentikelextraktion. Die lentikelextrahierenden Vorrichtungen und Verfahren haben den Vorteil, dass die Vorderfläche der Hornhaut in einem sehr viel kleineren Bereich verletzt wird. Man benötigt nicht mehr einen nahezu vollständig ringförmigen Einschnitt an der Hornhautvorderfläche, wie er zum Lösen einer das Hornhautinnere freilegenden Lamelle erforderlich ist. Es genügt vielmehr ein kleiner Einschnitt am Rand, der zum isolierten Volumen führt und durch den das isolierte Volumen entnommen werden kann, gegebenenfalls nach vorheriger Zerkleinerung des isolierten Materials. Das Prinzip der Lentikelextraktion erfordert es jedoch, die das Lentikel isolierenden Schnittflächen im Inneren der Hornhaut hochpräzise zu erzeugen. Um eine Fehlsichtigkeitskorrektur zu bewirken, sollte für einen möglichst geringen Gewebeverbrauch weiter mindestens eine der das Lentikel begrenzenden Schnittflächen in einem nicht konstanten Abstand zur Vorderfläche der Hornhaut liegen. Auch hier besteht ein Unterschied zum Ansatz, der eine Hornhautlamelle löst und abklappt, da dort die einzige und lamellenerzeugende Schnittfläche problemlos in konstantem Abstand zur Vorderfläche der Hornhaut liegen kann, also parallel zur Vorderfläche der Hornhaut. Wenn beim lamellenerzeugenden Schnitt die Vorderfläche der Hornhaut mit einem planen Kontaktglas flachgedrückt wird, muss man lediglich eine Schnittfläche erzeugen, die bis auf Randabschnitte parallel zur Fläche des Kontaktglases liegt und ihrerseits auch eben ist.

Die lentikelextrahierende Fehlsichtigkeitskorrektur ist grundlegend in der WO 2004/105660 und der WO 2004/105661 beschrieben. Im Stand der Technik sind darüber hinaus Weiterbildungen bekannt. So offenbart die WO 2005/011547 die Verwendung von Höhenlinien zur schnellen Lentikelisolation, die WO 2008/055697 gibt Berechnungsvorschriften, wie die Grenzflächen des Lentikels, d. h. die erzeugenden Schnittflächen gewählt werden können. Aus dieser Schrift ist es insbesondere bekannt, die das Lentikel begrenzenden Schnittflächen in eine anteriore Flap-Fläche, welche in konstantem Abstand zur Vorderfläche der Augenhornhaut liegt, und in eine posteriore Lentikelfläche aufzuteilen, welche in keinem konstanten Abstand zur Vorderfläche der Hornhaut liegt. Der Abstand der Flächen zueinander und damit ihre Form beeinflusst die Krümmung der Hornhaut nach der Korrektur.

Die WO 2008/055705 und WO 2008/055706 befassen sich mit der Problematik der Bildfeldkrümmung bei der Verwendung eines nicht planaren Kontaktglases und der Erzeugung von Steuerdaten für das chirurgische Verfahren.

Die Schnittfläche wird üblicherweise durch gepulste Laserstrahlung erzeugt. Dabei werden die Zielpunkte der Laserstrahlung entlang einer Bahnkurve angeordnet, die in der Schnittfläche liegt und letztlich die Schnittfläche vorgibt. Die WO 2008/055698 schildert die Anordnung der Zielpunkte entlang der Bahnkurve, wobei es vorgesehen ist, nicht für jeden in die Augenhornhaut abgegebenen Laserstrahlungspuls auch einen Zielpunkt vorzugeben.

Die WO 2008/131878 widmet sich der Frage, wie nach einem Abbruch eines laserchirurgischen Eingriffs, eine weitere Behandlung erfolgen kann, welche die bereits erzeugten Gewebsveränderungen in der Hornhaut berücksichtigt.

Die WO 2009/059711 und WO 2009/059730 befassen sich mit verschiedenen Profilen des zu entnehmenden Lentikels für bestimmte Fehlsichtigkeitskorrekturen, nämlich eine Hyperopiekorrektur, und geben Mindestwerte für das Lentikel vor.

Die WO 2003/059563 offenbart Betriebsparameter für eine Laservorrichtung zur operativen Fehlsichtigkeitskorrektur mittels Lentikelextraktion. Gleiches gilt für die EP 1628606 B1.

Für das lentikelextrahierende Verfahren ist im Stand der Technik das Femtosekunden-Laserkeratom VisuMax der Carl Zeiss Meditec AG bekannt. Es verwendet einen Femtosekunden-Faserlaser, welcher im infraroten Spektralbereich emittiert und mit einer Pulswiederholrate von 500 kHz Laserpulse abgibt, die in die Augenhornhaut fokussiert werden.

Sowohl für die Qualität einer Fehlsichtigkeitskorrektur als auch für die Akzeptanz bei Patienten ist die Zeit, welche die Schnittflächenerzeugung dauert, von hoher Bedeutung. Mit der Dauer des Eingriffs steigt das Risiko von störenden Augenbewegungen, welche die Genauigkeit der Schnittflächenerzeugung herabsetzen oder sogar dazu führen können, dass gar keine zusammenhängende Schnittfläche mehr erzeugt werden kann und das Verfahren abgebrochen werden muss. Auch ist ein länger dauernder Eingriff eine unerwünschte Belastung für den Patienten.

Bei der lamellenlösenden Fehlsichtigkeitskorrektur wird die Güte der optischen Korrektur durch die Genauigkeit des Volumenabtrages nach dem Lösen der Lamelle wesentlich bestimmt. Die Lage der lamellenlösenden Schnittfläche selbst ist von untergeordneter oder sogar verschwindender Bedeutung. Für die Güte des Resultates einer lentikelextrahierenden Sehkorrektur ist hingegen die exakte Positionierung der Schnittflächen in der Augenhornhaut von hoher Bedeutung. Da die Schnittfläche durch die Verstellung der Fokuslage (Fokus) der gepulsten Laserstrahlung entlang einer Bahn erzeugt wird, ist letztlich die Positioniergenauigkeit des Fokus in der Augenhornhaut wichtig. Hier darf nicht vergessen werden, dass es sich um lebendes Gewebe handelt, das sich mitunter während des Eingriffs verändern kann und auch nicht zwingend linear auf Parameterveränderungen reagiert.

Der Erfindung liegt deshalb die Aufgabe zugrunde, eine Vorrichtung der eingangs genannten Art bzw. ein Verfahren der eingangs genannten Art so weiterzubilden, dass damit eine schnelle Schnittflächenerzeugung bei gleichzeitig hoher Präzision erreicht wird.

Diese Aufgabe wird erfindungsgemäß gelöst durch eine Vorrichtung zum Isolieren eines Lentikels in der Hornhaut eines Auges, die aufweist
- eine Laserstrahlquelle, die ausgebildet ist, gepulste Laserstrahlung abzugeben mit
   - einer Pulsfrequenz von 1,2 MHz bis 10 MHz,
   - einer Pulsenergie von 1 nJ bis weniger als 100 nJ und
   - einer in die Hornhaut eindringenden Wellenlänge,
- einer Strahlformungseinrichtung, die aufweist
   - eine Strahloptik, welche ein Bildfeld hat und die gepulste Laserstrahlung in die Hornhaut in einen Fokus bündelt, der innerhalb des Bildfeldes liegt und einen maximalen Durchmesser unter 3 µm hat, und
   - eine Strahlablenkeinrichtung, die den Fokus in der Hornhaut und innerhalb des Bildfeldes verschiebt, wobei der Fokus bei ruhendem Bildfeld entlang einer Bahn wandert,
- eine Steuereinrichtung, die ausgebildet ist, die Laserstrahlquelle und die Strahlformungseinrichtung anzusteuern, um in der Hornhaut durch die Vorgabe der Bahn das Lentikel zu isolieren, welches durch mindestens eine Schnittfläche begrenzt ist, die gegenüber einer Vorderfläche der Hornhaut gekrümmt ist.

Die Aufgabe wird weiter gelöst mit einem Verfahren zum Isolieren eines Lentikels in der Hornhaut eines Auges, wobei
- in der Hornhaut mindestens eine Schnittfläche festgelegt wird, die das Lentikel umgrenzt und gegenüber einer Vorderfläche der Hornhaut gekrümmt ist,
- eine Bahn festgelegt wird, die in der Schnittfläche liegt,
- in die Hornhaut gepulste Laserstrahlung abgegeben wird mit
   - einer Pulsfrequenz von 1,2 MHz bis 10 MHz,
   - einer Pulsenergie von 1 nJ bis weniger als 100 nJ und
   - einer in die Hornhaut eindringenden Wellenlänge,
- eine Strahloptik verwendet wird, welche ein Bildfeld hat und die gepulste Laserstrahlung in die Hornhaut in einen Fokus bündelt, der innerhalb des Bildfeldes liegt und einen maximalen Durchmesser unter 3 µm hat, und
- der Fokus in der Hornhaut und innerhalb des Bildfeldes verschoben wird, wobei der Fokus bei ruhendem Bildfeld entlang der Bahn wandert,
- wobei das Verfahren keine chirurgische Behandlung des lebenden menschlichen oder tierischen Auges ausführt.

Die Erfindung kombiniert verschiedene Merkmale, die zusammen die Erzeugung einer Schnittfläche ermöglichen, welche gegenüber der Vorderfläche der Hornhaut gekrümmt ist. Es handelt sich also nicht um eine herkömmliche, lamellenlösende Schnittfläche, sondern um eine Schnittfläche, die in einer Schnittebene durch das Auge, welche die Sehachse oder die optische Achse des Auges enthält, nicht in konstantem Abstand zur Vorderfläche der Hornhaut verläuft. Die Krümmung, genauer gesagt handelt es sich um eine zweidimensionale Krümmung, gegenüber der Vorderfläche der Augenhornhaut ist bezüglich des erfindungsgemäßen Ansatzes auf einen zentralen Bereich um die optische Achse oder die Sehachse des Auges herum bezogen, der beispielsweise einen Durchmesser von nicht größer als 10 mm hat. Dies ist der für die optische Korrektur wesentliche Bereich, und in diesem Bereich haben übliche initiale Laserschnitte lamellenlösender Korrekturprinzipien einen konstanten Abstand zwischen Vorderfläche der Augenhornhaut und der Schnittfläche, welche die Lamelle zum Freilegen des Inneren der Augenhornhaut bewirkt.

Die erfindungsgemäße Kombination geht aus folgenden Gründen über eine einfache Aggregation von Merkmalen hinaus und erreicht eine Kombinationswirkung:
Die einzelnen Laserpulse, welche entlang der Bahn die Schnittfläche bilden, wirken in der Augenhornhaut unterschiedlich, je nach Pulsenergie, Pulsfrequenz und Fokusdurchmesser. Hinsichtlich der Gewebetrennung durch die gepulste Laserstrahlung können zwei unterschiedliche Arbeitsregime identifiziert werden. Diese unterschiedlichen Prozesse werden nachfolgend mit dem Begriff "Gewebespaltung" und "Gewebeschneiden" bezeichnet.

Fokussiert man Laserpulse unter bestimmten Bedingungen in die Hornhaut, kommt es im Fokusvolumen zu einer Gewebeauflösung, welche Gase freisetzt, die unter hohem Druck stehen und somit mechanische Kräfte auf die Gewebeumgebung ausüben. Das Hornhautgewebe besteht aus einer lamellenartigen Kollagenstruktur, sodass die mechanischen Kräfte nach Erkenntnis der Erfinder Mikrorupturen erzeugen, die entlang der Lamellen verlaufen. Somit entsteht zusätzlich zum primären Prozess der Gewebeauflösung im Fokusvolumen ein sekundärer Effekt, der zu einer Gewebespaltung entlang der Kollagenstruktur führt. Arbeitet man hingegen mit vergleichsweise geringeren Pulsenergien und höheren Repetitionsraten kommt es nicht mehr zum sekundären Effekt der Gewebespaltung, sondern lediglich die im Volumen des Fokus erzeugte Gewebeauflösung bewirkt das Trennen. Das Augenhornhautgewebe wird also weitgehend unabhängig von der Kollagenstruktur geschnitten.

Die Erfinder erkannten, dass zwischen Gewebespaltung und Gewebeschneiden kein abrupter Übergang erfolgt. Es gibt im Parameterraum der Laserpulsapplikation einen Übergangsbereich, in dem mal der eine oder der andere Mechanismus einen mehr oder weniger großen Anteil am Vorgang der Gewebetrennung hat. Weiter zeigte sich, dass in lentikelextrahierenden Verfahren und Vorrichtungen in der klinischen Praxis bislang ausschließlich Parametersätze angewendet wurden, die dazu führten, dass der sekundäre Effekt der Gewebespaltung wesentlichen Anteil am Trennprozess hat. Eine Verwendung ausschließlich gewebeschneidender Parametersätze erfolgt derzeit in der klinischen Praxis ausschließlich im Laserkeratom der Ziemer Ophthalmie Systems AG, Port, Schweiz. Diese Vorrichtung ist jedoch zur Erzeugung von Schnittflächen, welche gegenüber der Vorderfläche der Hornhaut gekrümmt sind, nach öffentlichem Kenntnisstand nicht in der Lage, und das Arbeitsregime bietet, wie bereits eingangs ausgeführt, beim lamellenlösenden Verfahren keinen wesentlichen applikativen Vorteil und ist daher nicht von Bedeutung.

Es muss betont werden, dass die Identifikation der unterschiedlichen Arbeitsregime, d. h. der Parametereinfluss auf die Frage, ob Gewebespaltung oder Gewebeschneiden bei der Lentikelerzeugung überwiegt, erstmals von den Erfindern genannt wurde. Im Stand der Technik findet sich die Unterscheidung der Arbeitsregime bei diesem Verfahren nicht. Auch die Zuordnung der einzelnen Realisierungen des Standes der Technik zu diesen Arbeitsregimen kennt die Literatur nicht.

Die Erfinder erkannten durch Ihre Arbeiten weiter, dass hinsichtlich der zu erreichenden Genauigkeit ein wesentlicher Unterschied zwischen Gewebespaltung einerseits und Gewebeschneiden andererseits genau nur dann besteht, wenn gekrümmte Schnitte durchgeführt werden. Denn die Gewebespaltung verläuft in der Regel immer entlang der Lamellen der Kollagenstruktur des Hornhautgewebes. Bei lamellären Schnitten besteht daher im Bearbeitungsergebnis kein Vor-oder Nachteil zwischen einer lamellären Spaltung und einem lamellären Schnitt. Anders bei gekrümmten Schnittflächen: Denn aufgrund der physiologisch gegebenen Lamellendicke und -struktur ist es nicht möglich, eine gekrümmte Schnittfläche mit hoher Formtreue an eine beliebige Stelle in der Augenhornhaut zu plazieren, wenn mit gewebespaltender Trennung gearbeitet wird.

Mit dem Mechanismus des Gewebeschneidens kann eine sehr viel genauere Schnittflächenpositionierung und insbesondere eine höhere Formtreue erreicht werden. Die Erfinder führen dies darauf zurück, dass bei einem gewebeschneidenden Prozess anders als bei der Gewebespaltung die Schnittfläche nicht zwingend in Grenzflächen zwischen Lamellen der Kollagenstruktur des Hornhautgewebes verläuft, sondern auch innerhalb einer Lamelle angeordnet sein kann. Ein bei der Gewebespaltung in der Lamellenebene erfolgender Vorriss (Voreilen der Spaltung vor der fortschreitenden Laserpulsfolge) wird vermieden und die Trennung erfolgt genau in der Fokusposition. Die Gewebespaltung hingegen kann quasi als Digitalisierung der Schnittflächenposition verstanden werden, wobei die kleinste Einheit die Lamellendicke ist, da eine gewebespaltend erzeugte Schnittfläche dazu tendiert, immer in Grenzflächen zwischen einzelnen Lamellen des Hornhautgewebes zu liegen. Dies ist für Schnitte, die in konstantem Abstand zur Hornhautvorderseite erfolgen kein Mangel, denn die Lamellenstruktur folgt der Hornhautvorderseite mit guter Genauigkeit. Für gekrümmte Lentikelschnittflächen, deren Abstand zur Hornhautvorderseite je nach Radius und ggf. Winkel variiert, erweist sich diese Art der Schnittflächenerzeugung aber als Nachteil.

Diese Zusammenhänge führen dazu, dass der erfindungsgemäße Parameterbereich hinsichtlich Pulsfrequenz, Pulsenergie und Fokusdurchmesser besonders zuverlässig zu einer Gewebetrennung führt, bei der der Vorgang des Gewebeschneidens weit überwiegt. Die Kombination mit der Erzeugung einer Schnittfläche, welche gegenüber der Vorderfläche der Hornhaut gekrümmt ist und damit zwangsläufig nicht der Grenzfläche zwischen Lamellen der Kollagenstruktur des Hornhautgewebes (welche im übrigen weitgehend parallel zur Hornhautvorderfläche verläuft) folgt, führt deshalb zu einer besonders genauen Schnittflächenpositionierung und hohen Formtreue. Im erfindungsgemäßen Arbeitsbereich ist der Beitrag der Gewebespaltung zugunsten der Wirkung des Gewebeschneidens reduziert, sodass für die Lentikelextraktion die Schnittfläche und damit die Isolation des Lentikels präziser den vorgegebenen Werten folgen.

Der gewebeschneidende Effekt ist besonders groß, da die Pulsenergie weniger als 100 nJ und, besonders bevorzugt, weniger als 10 nJ beträgt. Als besonders günstig hat sich ein Pulsenergiebereich von 10 nJ bis 80 nJ herausgestellt. Gleiches gilt für die Pulslänge der gepulsten Laserstrahlung, die bei infraroten Wellenlängen nicht mehr als 1 ps betragen sollte.

Die Wellenlänge der verwendeten Laserstrahlung ist so, dass die Laserstrahlung in die Augenhornhaut eindringen kann (Transmissionsgrad ≥ 0,8) und dort mittels nichtlinearer Effekte im Inneren der Hornhaut zur Gewebetrennung führt. Hierfür hat sich eine Wellenlänge von 1030-1060 nm herausgestellt. Alternativ kann auch Laserstrahlung im ultravioletten Spektralbereich zwischen 300 nm und 400 nm eingesetzt werden. Sie hat zwar tendenziell einen höheren linearen Wechselwirkungsanteil (Absorption) als die genannten infrarote Strahlung, ist aber ebenfalls geeignet, wenn der Fokusdurchmesser 2 µm nicht überschreitet und die Pulsfrequenz nicht über 2 MHz liegt. Wenn Laserpulse aus diesem ultravioletten Wellenlängenbereich verwendet werden, können auch Pulslängen von einigen ns wirksam eingesetzt werden.

Die Isolierung des Lentikels erfolgt dadurch, dass der Fokus innerhalb des Bildfeldes der verwendeten Strahloptik verschoben wird und das Bildfeld gegenüber der zu bearbeitenden Hornhaut ruht. Hierin besteht ein Unterschied zum Laserkeratom der Ziemer Ophthalmie Systems AG, bei der ein Mikroskopobjekt zur Anwendung kommt, dessen Bildfeld viel zu klein ist, um den in der Hornhaut abzuarbeitenden Bereich vollständig zu erfassen. Das Mikroskopobjekt und damit das Bildfeld wird deshalb bei diesem Laserkeratom verschoben. Eine gekrümmte Schnittfläche kann damit nicht mit vertretbarem Aufwand erzeugt werden, weshalb dieses Laserkeratom die Vorderfläche der Augenhornhaut durch ein planares Kontaktglas flachdrückt, sodass die erzeugte Schnittfläche automatisch parallel zur Augenhornhautvorderfläche liegt. Die Erfindung verwendet hingegen ein ruhendes Bildfeld, für das im Hinblick auf den zu bearbeitenden Bereich ein Durchmesser von mindestens 3 mm, bevorzugt mindestens 6 mm und besonders bevorzugt mindestens 7 mm sinnvoll ist. Kleinere Bildfelder erschweren die Durchführung der Schnittflächenerzeugung bzw. sind für übliche zu isolierende Lentikel zu klein.

Da die Schnittfläche durch Verschieben des Fokus innerhalb des Bildfeldes entlang einer Bahnkurve erfolgt, die in der Schnittfläche liegt, kann bei geeigneter dreidimensionaler Fokuslagenverstellung nahezu jede beliebige gekrümmte Schnittfläche in der Augenhornhaut erzeugt werden. Es ist deshalb nicht mehr erforderlich, die Augenhornhaut flachzudrücken. Vielmehr kann mit einem gekrümmten Kontaktglas gearbeitet werden, wobei es zu bevorzugen ist, dass eine auf die Vorderfläche der Hornhaut aufzusetzende Kontaktfläche dieses Kontaktglases einen Krümmungsradius von nicht über 50 mm, bevorzugt nicht über 20 mm hat. Die Krümmung der Kontaktfläche des Kontaktglases gibt die Krümmung der Hornhaut während des Eingriffs vor.

Für die Erzeugung der gekrümmten Schnittfläche ist es weiter vorteilhaft, wenn die Strahloptik ein Objektiv mit numerischer Apertur von mindestens 0,33 in der Hornhaut aufweist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Insbesondere ist die Erfindung in leicht abgewandelter Form auch für die Erzeugung von gekrümmten Schnitten in anderen Elementen des Auges vorteilhaft anwendbar, beispielsweise in der Linse oder dem Glaskörper.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Schemadarstellung einer Vorrichtung für die Durchführung einer lentikelextrahierenden Fehlsichtigkeitskorrektur,
- Fig. 2: eine vereinfachte Schnittdarstellung durch die Augenhornhaut zur Veranschaulichung des zu isolierenden und extrahierenden Lentikels und
- Fig. 3: eine Projektion einer das Lentikel begrenzenden Schnittfläche zur Verdeutlichung der Schnittflächenerzeugung.

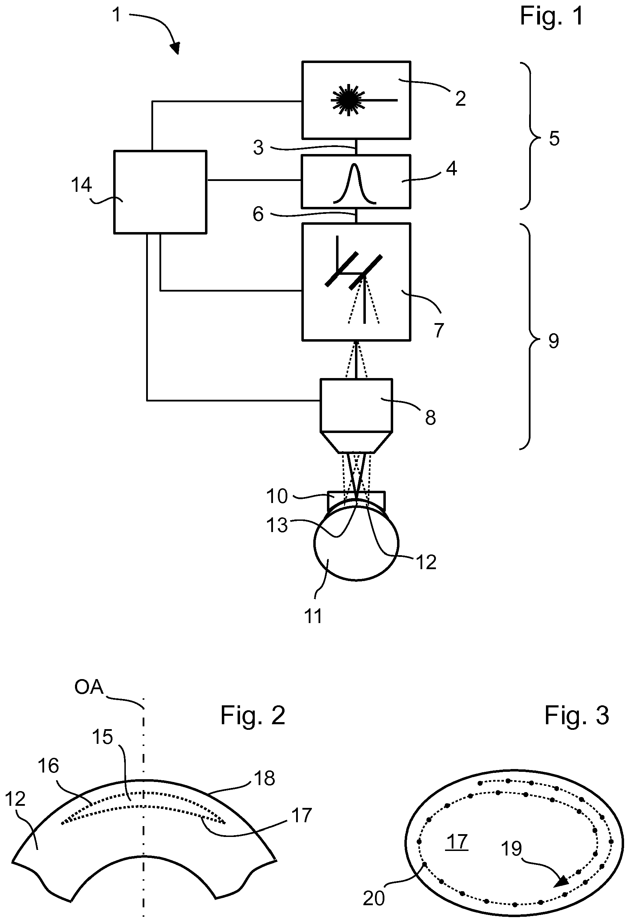

Fig. 1 zeigt schematisch eine Vorrichtung 1 zum Ausführen einer lentikelextrahierenden Fehlsichtigkeitskorrektur. Die Vorrichtung 1 weist einen Laser 2 auf, der gepulste Laserstrahlung bereitstellt, wobei in der geschilderten Ausführungsform der Laser 2 einen gepulsten Rohstrahl 3 abgibt, der eine Wellenlänge hat, welche in das Gewebe der Augenhornhaut eindringt, sodass dort eine Bearbeitung mittels nichtlinearer Effekte erfolgen kann. Der Rohstrahl 3 wird von einem Pulsformer 4 hinsichtlich der Pulsdauer geformt, wobei eine aus dem Stand der Technik bekannte Vorverzerrung erfolgen kann, die dafür sorgt, dass nach Durchlauf durch den weiteren optischen Weg des Strahlengangs im Material, d. h. in der Hornhaut des Auges die gewünschte Pulslänge von beispielsweise ≤ 1 ps vorliegt. Der Pulsformer 4 und der Laser 2 bilden zusammen eine Laserstrahlquelle 5, welche einen gepulsten Laserstrahl 6 der gewünschten Pulslänge abgibt.

Der gepulste Laserstrahl 6 fällt weiter durch einen Scanner 7, welcher bevorzugt eine zweidimensionale Ablenkung quer zur Ausbreitungsrichtung der Laserstrahlung bewirkt. Der derart gescannte Laserstrahl 6 wird von einem Objektiv 8 in die Augenhornhaut fokussiert. Der Scanner 7 bildet zusammen mit dem Objektiv 8 eine Strahlformungseinrichtung 9, die dafür sorgt, dass die gepulste Laserstrahlung 6 an einstellbare Orte in eine Hornhaut eines Auges fokussiert wird, wobei der Fokusdurchmesser dort weniger als 3 µm beträgt.

Die Laserstrahlquelle 5 kann optional einen sogenannten Pulspicker umfassen, der beispielsweise Bestandteil des Pulsformers 4 sein kann. Dieser Pulspicker verändert den Rohstrahl 3 hinsichtlich der Frequenz der Laserstrahlungspulse, die in der Augenhornhaut einen Bearbeitungseffekt haben. So ist es beispielsweise möglich, den Laser 2 so zu auszubilden, dass er einen Rohstrahl 3 mit einer Pulsfrequenz bereitstellt, die deutlich höher ist, als diejenige Pulsfrequenz, die für die bearbeitungswirksamen Laserpulse des Laserstrahls 6 gewünscht ist. Der Pulspicker reduziert dann die Frequenz der wirksamen Laserstrahlungspulse, indem er einzelne Laserstrahlungspulse hinsichtlich ihrer Bearbeitungswirkung unschädlich macht. Dies kann beispielsweise dadurch erfolgen, dass der Pulspicker die Pulslänge vergrößert. Die Bedeutung des Pulspickers, der im Stand der Technik bekannt ist, wird später noch anhand der Fig. 3 erläutert werden.

In der in Fig. 1 gezeigten Ausführungsform beträgt die Pulsfrequenz des gepulsten Laserstrahls 6 zwischen 1,2 MHz und 10 MHz, wobei die Frequenz auf diejenigen Pulse bezogen sind, die einen Bearbeitungseffekt haben, d. h. Pulse, die nicht von einem gegebenenfalls vorhandenen Pulspicker unschädlich gemacht wurden.

Die Energie dieser Pulse des gepulsten Laserstrahls 6 liegt zwischen 1 nJ und weniger als 100 nJ, bevorzugt zwischen 10 nJ und weniger als 100 nJ, besonders bevorzugt zwischen 20 nJ und 80 nJ.

Die Wellenlänge des Laserstrahls 6 liegt in einem Bereich von 1030 nm bis 1060 nm oder von 300 nm bis 400 nm oder einem anderen Spektralbereich, der in die Hornhaut eindringen kann, für den die Hornhaut also einen Transmissionsgrad von mindestens 0,8 hat.

Die Vorrichtung 1 umfasst weiter ein Kontaktglas 10, das zur Fixierung des Auges 11 dient und auch dazu, um einer Vorderfläche der Hornhaut 12 des Auges 11 eine gewünschte und bekannte Form zu verleihen. Die entsprechende Kontaktfläche des Kontaktglases 10 hat dazu einen Krümmungsradius von 50 mm oder kleiner, besonderes bevorzugt 20 mm oder kleiner. Das Objektiv 8 bündelt die Laserstrahlung 6 in einen Fokus 13, der innerhalb der Hornhaut 12 liegt. Der Fokus 13 hat einen maximalen Durchmesser von 3 µm, bevorzugt von maximal 2 µm. Der maximale Durchmesser ist dabei der größte Durchmesser, der z. B. im Falle eines elliptischen Fokusflecks entlang der großen Halbachse gemessen wird. Im Falle eines kreisförmigen Spots ist der Spot-Durchmesser das relevante Maß.

Fig. 1 zeigt gestrichelt, dass je nach Wirkung des Scanners 7 der Fokus 13 an verschiedenen Stellen in der Hornhaut 12 des Auges 11 liegt. Der Scanner 7 bewirkt in der Bauweise der Fig. 1 eine Ablenkung quer zur Haupteinfallsrichtung der Laserstrahlung 6. Eine Verstellung der Fokusposition längs der Haupteinfallsrichtung erfolgt durch geeignete Ansteuerung des Objektivs 8, das für eine z-Verstellung geeignet ausgebildet ist.

Die Laserstrahlquelle 5 (in der Bauweise der Fig. 1 realisiert durch Laser 2 und Pulsformer 4) sowie die Strahlformungseinrichtung 9 (in der Bauweise der Fig. 1 realisiert durch Scanner 7 und Objektiv 8) sind über nicht weiter bezeichnete Steuerleitungen mit einem Steuergerät 14 verbunden, das diese Elemente geeignet ansteuert. Das Steuergerät 14 erzeugt durch die Ansteuerung eine Schnittfläche in der Augenhornhaut. Die entsprechenden Verhältnisse sind in einer Schnittdarstellung in Fig. 2 dargestellt, welche schematisch die Hornhaut 12 zeigt.

Durch die Verstellung des Fokus 13 der gepulsten Laserstrahlung 6 wird ein Lentikel 15 in der Hornhaut 12 isoliert. Das Lentikel 15 ist anterior durch eine Flap-Fläche 16 und posterior durch eine Lentikel-Fläche 17 begrenzt. Um die Grenzflächen des Lentikels 15 möglichst einfach halten zu können, befindet sich die Flap-Fläche 16 in konstantem Abstand zur Vorderfläche 18 der Hornhaut 12. Die Flap-Fläche 16 ist somit nicht gegenüber der Vorderfläche 18 gekrümmt. Dies ist bei der Lentikel-Fläche 17 anders, die gegenüber der Vorderfläche 18 gekrümmt ist. Ohne eine solche Krümmung würde die Entnahme des Lentikels 15 die Krümmung der Vorderfläche 18 der Hornhaut 12 nicht ändern. Die gegenüber der Vorderfläche 18 gekrümmte Lentikel-Fläche 17 stellt hingegen eine Änderung der Krümmung der Vorderfläche 18 der Hornhaut 12 ein, wenn das Lentikel 15 entnommen wird. Diese Entnahme geschieht durch einen in der Fig. 2 nicht dargestellten seitlichen Schnitt, der beispielsweise am Rand des Lentikels 15 von der Flap-Fläche 16 zur Vorderfläche 18 führt und es erlaubt, das isolierte Lentikel 15 zu extrahieren, gegebenenfalls nach vorheriger Zerkleinerung des Materials des Lentikels 15. In der Darstellung der Fig. 2 sind Flap-Fläche 16 und Lentikel-Fläche 17 symmetrisch zur optischen Achse OA. Dies stellt sich für die Flap-Fläche 16 automatisch ein, wenn sie konstanten Abstand zur Vorderfläche 18 hat.

Die Grenzflächen des Lentikels 15 können außer der Flap-Fläche 16 und der Lentikel-Fläche 17 natürlich noch weitere Flächen umfassen. Beispielsweise kann bei einem Lentikel 15, das auf der optischen Achse OA dünner ist, als in achsenfernen Bereichen, eine zusätzliche Randfläche vorgesehen sein, welche die Flap-Fläche 16 mit der Lentikel-Fläche 17 verbindet, die dann einen stärker gekrümmten Verlauf aufweist, als die Flap-Fläche 16 und die Vorderfläche 18.

Die Schnittflächen zum Isolieren des Lentikels 15 werden dadurch erzeugt, dass der Fokus 13 entlang einer Bahn verschoben wird, die in der entsprechenden Fläche liegt. Dies ist exemplarisch in Fig. 3 anhand der Lentikel-Fläche 17 gezeigt, die aus Anschaulichkeitsgründen hier elliptisch ist. Dies soll zeigen, dass mit der Vorrichtung 1 nicht nur ein sphärischer Sehfehler, sondern auch ein Astigmatismus korrigiert werden kann. Grundsätzlich ist bei der Korrektur höherer Abberationen das Lentikel 15 nicht mehr rotationssymmetrisch zur optischen Achse OA. Fig. 3 zeigt eine Auffaltung der Lentikel-Fläche 17 in die Zeichnungsebene. Gestrichelt ist in Fig. 3 eine Bahn 19 eingetragen. Entlang dieser Bahn wird die Position des Fokus 13 verstellt. Dabei ist natürlich in der Regel nicht nur eine Verstellung quer zur optischen Achse OA nötig, sondern auch eine Verstellung der Fokusposition längs der optischen Achse OA. Dies ist in Fig. 3 deshalb nicht zu erkennen, da diese Figur eine Auffaltung der Lentikel-Fläche 17 in die Zeichnungsebene zeigt, weshalb die Bahn 19 in der Darstellung der Fig. 3 in einer Ebene liegt. Betrachtet man sich den Schnitt durch das Lentikel 15 in Fig. 2, wird deutlich, dass mit zunehmendem Abstand von der optischen Achse OA auch die z-Position des Fokus von der Vorderfläche 18 hin weggeschoben wird.

Entlang der Bahn 19 der Fig. 3 sind Zielpunkte 20 eingetragen. Sie bezeichnen jeweils einen Punkt, auf den ein Laserpuls der gepulsten Laserstrahlung 6 abgegeben wird. Durch Aneinanderreihung der Zielpunkte 20 entlang der Bahn 19 und durch geeignete Wahl der Bahn 19 wird insgesamt die Lentikel-Fläche 17 als Schnittfläche ausgebildet. Die Abstände der Zielpunkte 20 sind dabei so gewählt, dass möglichst keine Materialbrücken stehen bleiben, die Lentikel-Fläche 17 also vollflächig als Schnittfläche generiert ist.

Anhand Fig. 3 ist leicht zu verstehen, warum es vorteilhaft ist, die Pulsfrequenz des gepulsten Laserstrahls 6 veränderbar zu machen. Möchte man die Zielpunkte 20 möglichst äquidistant anordnen, sind die Pulsfrequenz und die Verschiebgeschwindigkeit der Stahlformungseinrichtung 9 aufeinander anzupassen. Da ein Laser 2 bei hoher Pulsfrequenz in der Regel nur unter großem Aufwand verstellbar ist, ist es von Vorteil, mit dem Laser 2 zuerst einen Rohstrahl 3 bereitzustellen, der eine Pulsfrequenz hat, welche größer gleich der maximalen Pulsfrequenz ist, die für den Laserstrahl 6 gewünscht wird. Es kann einfacher sein, einen solchen Laser 2 zu realisieren und mit einem Pulspicker zu kombinieren, als einen Laser aufzubauen, der hinsichtlich seiner Pulsfrequenz direkt verstellbar ist. So kann die Pulsfrequenz an die Verschiebegeschwindigkeit angepasst werden und man minimiert die Dauer der Schnittflächenerzeugung.

Die in dieser Figurenbeschreibung sowie im allgemeinen Teil der Beschreibung genannten Parameter für Pulsenergie, Pulsfrequenz, Fokusdurchmesser und optional Pulsdauer führen dazu, dass die Schnittflächen mit einem Mechanismus der Gewebetrennung erzeugt werden, der das Gewebeschneiden und im wesentlichen die Gewebespaltung ausnützt. Damit kann eine gewünschte Lage für die begrenzenden Schnittflächen des Lentikels 15 hochgenau realisiert werden.

## Patentansprüche

1. Vorrichtung zum Isolieren eines Lentikels (15) in der Hornhaut (12) eines Auges (11), die aufweist
- eine Laserstrahlquelle (5), die ausgebildet ist, gepulste Laserstrahlung (6) abzugeben mit
- einer Pulsfrequenz von 1,2 MHz bis 10 MHz,
- einer Pulsenergie von 1 nJ bis weniger als 100 nJ und
- einer in die Hornhaut (12) eindringenden Wellenlänge,
- einer Strahlformungseinrichtung (9), die aufweist
- eine Strahloptik (8), welche ein Bildfeld hat und die gepulste Laserstrahlung (6) in die Hornhaut (12) in einen Fokus (13) bündelt, der innerhalb des Bildfeldes liegt und einen maximalen Durchmesser unter 3 µm hat, und
- eine Strahlablenkeinrichtung (7), die den Fokus (13) in der Hornhaut (12) und innerhalb des Bildfeldes verschiebt, wobei der Fokus (13) bei ruhendem Bildfeld entlang einer Bahn (19) wandert,
- eine Steuereinrichtung (14), die ausgebildet ist, die Laserstrahlquelle (5) und die Strahlformungseinrichtung (9) anzusteuern, um in der Hornhaut (12) durch die Vorgabe der Bahn (19) das Lentikel zu isolieren, welches durch mindestens eine Schnittfläche (16, 17) begrenzt ist, die gegenüber einer Vorderfläche (18) der Hornhaut (12) gekrümmt ist.

2. Vorrichtung nach Anspruch 1, wobei die Laserstrahlquelle (5) ausgebildet ist, die gepulste Laserstrahlung (6) mit einer Pulsenergie von 10 nJ oder mehr abzugeben und/oder wobei die Laserstrahlquelle (5) ausgebildet ist, die gepulste Laserstrahlung (6) mit einer Pulsenergie von 10 nJ bis 80 nJ abzugeben.

3. Vorrichtung nach einem der obigen Ansprüche, wobei die Laserstrahlquelle (5) ausgebildet ist, die gepulste Laserstrahlung (6) mit einer Wellenlänge von 1030 nm bis 1060 nm abzugeben.

4. Vorrichtung nach Anspruch 3, wobei die Laserstrahlquelle (5) ausgebildet ist, die gepulste Laserstrahlung (6) mit einer Pluslänge unter 1 ps abzugeben.

5. Vorrichtung nach einem der Ansprüche 1 bis 3, wobei die Laserstrahlquelle (5) ausgebildet ist, die gepulste Laserstrahlung (6) mit einer Wellenlänge von 300 nm bis 400 nm und einer Pulsfrequenz nicht über 10 MHz abzugeben, und die Strahloptik die gepulste Laserstrahlung in den Fokus (13) bündelt, der einen maximalen Durchmesser kleiner oder gleich 2 µm hat.

6. Vorrichtung nach einem der obigen Ansprüche, die ein Kontaktglas (10) zum Aufsetzen auf die Hornhaut (12) aufweist, wobei eine auf die Vorderfläche (18) der Hornhaut (12) aufzusetzende Kontaktfläche des Kontaktglases (10) einen Krümmungsradius nicht über 50 mm, bevorzugt nicht über 20 mm hat.

7. Vorrichtung nach einem der obigen Ansprüche, wobei die Strahloptik ein Objektiv (8) mit numerischer Apertur von mindestens 0,33 aufweist.

8. Vorrichtung nach einem der obigen Ansprüche, wobei die Strahloptik (8) in der Hornhaut (12) des Auges (11) ein optisches Feld mit einem Durchmesser von ≥ 3 mm, insbesondere von ≥ 6 mm und besonders bevorzugt von ≥ 7 mm hat.

9. Verfahren zum Isolieren eines Lentikels (15) in der Hornhaut (12) eines Auges (11), wobei
- in der Hornhaut (12) mindestens eine Schnittfläche (16, 17) festgelegt wird, die das Lentikel (15) umgrenzt und die nicht in einem konstanten Abstand zur Vorderfläche (18) der Hornhaut (12) verläuft,
- eine Bahn (19) festgelegt wird, die in der Schnittfläche (16, 17) liegt,
- in die Hornhaut (12) gepulste Laserstrahlung (6) abgegeben wird mit
- einer Pulsfrequenz von 1,2 MHz bis 10 MHz,
- einer Pulsenergie von 1 nJ bis weniger als 100 nJ und
- einer in die Hornhaut (12) eindringenden Wellenlänge,
- eine Strahloptik (8) verwendet wird, welche ein Bildfeld hat und die gepulste Laserstrahlung (6) in die Hornhaut (12) in einen Fokus (13) bündelt, der innerhalb des Bildfeldes liegt und einen maximalen Durchmesser unter 3 µm hat, und
- der Fokus (13) in der Hornhaut (12) und innerhalb des Bildfeldes verschoben wird, wobei der Fokus (13) bei ruhendem Bildfeld entlang der Bahn (19) wandert,
- wobei das Verfahren keine chirurgische Behandlung des lebenden menschlichen oder tierischen Auges ausführt.

10. Verfahren nach Anspruch 9, wobei die Pulsenergie einen Wert von 10 nJ oder mehr hat und/oder wobei die Pulsenergie einen Wert von 10 nJ bis 80 nJ hat.

11. Verfahren nach einem der Ansprüche 9 oder 10, wobei die Wellenlänge einen Wert von 1030 nm bis 1060 nm hat, wobei optional die Pluslänge unter 1 ps liegt.

12. Verfahren nach einem der Ansprüche 9 bis 11, wobei die Wellenlänge einen Wert von 300 nm bis 400 nm, die Pulsfrequenz einen Wert nicht über 2 MHz und der Fokus einen maximalen Durchmesser kleiner oder gleich 2 µm hat.

13. Verfahren nach einem der Ansprüche 9 bis 12, wobei ein Kontaktglas (10) auf das Auge aufgesetzt wird, das eine auf der Vorderfläche (18) der Hornhaut (12) aufgesetzte Kontaktfläche mit einem Krümmungsradius nicht über 50 mm, bevorzugt nicht über 20 mm hat.

14. Verfahren nach einem der Ansprüche 9 bis 13, wobei die Strahloptik (8) eine numerische Apertur von mindestens 0,33 hat.

15. Verfahren nach einem der Ansprüche 9 bis 14, wobei die Strahloptik (8) in der Hornhaut (12) des Auges (11) ein optisches Feld mit einem Durchmesser von ≥ 3 mm, insbesondere von ≥ 6 mm und besonders bevorzugt von ≥ 7 mm hat.

## Claims

1. Device for isolating a lenticule (15) in the cornea (12) of an eye (11), which comprises
- a laser beam source (5) that is configured to emit pulsed laser radiation (6) with
- a pulse frequency of 1.2 MHz to 10 MHz,
- a pulse energy of 1 nJ to less than 200 nJ and
- a wavelength which penetrates the cornea (12),
- a beam forming unit (9) which comprises
- beam optics (8), which have an image field and which bundle the pulsed laser radiation (6) in the cornea (12) into a focus (13) which is located inside the image field and which has a maximum diameter of less than 3 µm, and
- a beam deflection unit (7) which shifts the focus (13) in the cornea (12) and inside the image field, wherein the focus (13) moves along a path (19) at resting image field,
- a control unit (14), which is configured to control the laser beam source (5) and the beam forming unit (9), in order to isolate the lenticule in the cornea (12) by specifying the path (19), which lenticule is delimited by at least one cut surface (16, 17) which is curved with regard to a front surface (18) of the cornea (12).

2. Device according to claim 1, wherein the laser beam source (5) is configured to emit the pulsed laser radiation (6) with a pulse energy of 10 nJ or more and/or wherein the laser beam source (5) is configured to emit the pulsed laser radiation (6) with a pulse energy of 10 nJ to 80 nJ.

3. Device according to one of the previous claims, wherein the laser beam source (5) is configured to emit the pulsed laser radiation (6) with a wavelength of 1030 nm to 1060 nm.

4. Device according to claim 3, wherein the laser beam source (5) is configured to emit the pulsed laser radiation (6) with a pulse length of less than 1 ps.

5. Device according to one of claims 1 to 3, wherein the laser beam source (5) is configured to emit the pulsed laser radiation (6) with a wavelength of 300 nm to 400 nm and a pulse frequency of not more than 10 MHz, and the beam optics bundle the pulsed laser radiation into the focus (13), which focus has a maximum diameter of less than or equal to 2 µm.

6. Device according to one of the previous claims, comprising a contact lens (10) for placing on the cornea (12), wherein a contact surface of the contact lens (10) to be placed on the front surface (18) of the cornea (12) has a radius of curvature of not more than 50 mm, preferably not more than 20 mm.

7. Device according to one of the previous claims, wherein the beam optics comprise an objective (8) with a numerical aperture of at least 0.33.

8. Device according to one of the previous claims, wherein, in the cornea (12) of the eye (11), the beam optics (8) have an optical field with a diameter of ≥ 3 mm, in particular of ≥ 6 mm and particularly preferably of ≥ 7 mm.

9. Method for isolating a lenticule (15) in the cornea (12) of an eye (11), wherein
- at least one cut surface (16, 17) is defined in the cornea (12), which surface circumscribes the lenticule (15) and which does not travel at a uniform distance from the front surface (18) of the cornea (12),
- a path (19) which lies in the cut surface (16, 17) is defined,
- pulsed laser radiation (6) is emitted into the cornea (12) with
- a pulse frequency of 1.2 MHz to 10 MHz,
- a pulse energy of 1 nJ to less than 100 nJ and
- a wavelength which penetrates the cornea (12),
- beam optics (8) are used, which optics have an image field and which bundle the pulsed laser radiation (6) in the cornea (12) into a focus (13) which is located inside the image field and which has a maximum diameter of less than 3 µm, and
- the focus (13) is shifted in the cornea (12) and within the image field, wherein the focus (13) moves along the path (19) at resting image field,
- wherein the method does not carry out surgical treatment or the living human or animal eye.

10. Method according to claim 9, wherein the pulse energy has a value of 10 nJ or more and/or wherein the pulse energy has a value of 10 nJ to 80 nJ.

11. Method according to one of claims 9 or 10, wherein the wavelength has a value of 1030 nm to 1060 nm, wherein the pulse length is optionally less than 1 ps.

12. Method according to one of claims 9 to 11, wherein the wavelength has a value of 300 nm to 400 nm, the pulse frequency has a value of not more than 2 MHz and the focus has a maximum diameter of less than or equal to 2 µm.

13. Method according to one of claims 9 to 12, wherein a contact lens (10) is placed on the eye, which contact lens has a contact surface placed on the front surface (18) of the cornea (12) with a radius of curvature of not more than 50 mm, preferably not more than 20 mm.

14. Method according to one of claims 9 to 13, wherein the beam optics (8) have a numerical aperture of at least 0.33.

15. Method according to one of claims 9 to 14, wherein, in the cornea (12) of the eye (11), the beam optics (8) have an optical field with a diameter of ≥ 3 mm, in particular of ≥ 6 mm and particularly preferably of ≥ 7 mm.

## Revendications

1. Arrangement pour isoler une lenticule (15) dans la cornée (12) d'un œil (11), comprenant
- une source de faisceau laser (5), qui est configurée pour délivrer un rayonnement laser pulsé (6) avec
- une fréquence d'impulsions de 1,2 MHz à 10 MHz,
- une énergie d'impulsion de 1 nJ à moins de 100 nJ et
- une longueur d'onde qui pénètre dans la cornée (12),
- un dispositif de mise en forme de faisceau (9), qui possède
- une optique de rayonnement (8), qui comprend un champ d'image et qui focalise le rayonnement laser pulsé (6) dans la cornée (12) en un foyer (13) qui se trouve à l'intérieur du champ d'image et présente un diamètre maximal inférieur à 3 µm, et
- un dispositif de déviation de faisceau (7) qui décale le foyer (13) dans la cornée (12) et à l'intérieur du champ d'image, le foyer (13) migrant le long d'une trajectoire (19) lorsque le champ d'image est au repos,
- un dispositif de commande (14), qui est configuré pour commander la source de faisceau laser (5) et le dispositif de mise en forme de faisceau (9) afin d'isoler la lenticule dans la cornée (12) par prédéfinition de la trajectoire (19), la lenticule étant délimitée par au moins une surface de coupe (16, 17) qui est incurvée par rapport à une surface avant (18) de la cornée (12).

2. Arrangement selon la revendication 1, la source de faisceau laser (5) étant configurée pour délivrer le rayonnement laser pulsé (6) avec une énergie d'impulsion égale ou supérieure à 10 nJ et/ou la source de faisceau laser (5) étant configurée pour délivrer le rayonnement laser pulsé (6) avec une énergie d'impulsion de 10 nJ à 80 nJ.

3. Arrangement selon l'une des revendications précédentes, la source de faisceau laser (5) étant configurée pour délivrer le rayonnement laser pulsé (6) avec une longueur d'onde de 1030 nm à 1060 nm.

4. Arrangement selon la revendication 3, la source de faisceau laser (5) étant configurée pour délivrer le rayonnement laser pulsé (6) avec une longueur d'impulsion inférieure à 1 ps.

5. Arrangement selon l'une des revendications 1 à 3, la source de faisceau laser (5) étant configurée pour délivrer le rayonnement laser pulsé (6) avec une longueur d'onde de 300 nm à 400 nm et une fréquence d'impulsions non supérieure à 10 MHz, et l'optique de rayonnement focalisant le rayonnement laser pulsé en le foyer (13) qui présente un diamètre maximal inférieur ou égal à 2 µm.

6. Arrangement selon l'une des revendications précédentes, lequel possède un verre de contact (10) destiné à être posé sur la cornée (12), une surface de contact du verre de contact (10), à poser sur la surface avant (18) de la cornée (12) présentant un rayon de courbure non supérieur à 50 mm, de préférence non supérieur à 20 mm.

7. Arrangement selon l'une des revendications précédentes, l'optique de rayonnement possédant un objectif (8) ayant une ouverture numérique d'au moins 0,33.

8. Arrangement selon l'une des revendications précédentes, l'optique de rayonnement (8) possédant dans la cornée (12) de l'œil (11) un champ optique ayant un diamètre ≥ 3 mm, notamment ≥ 6 mm et notamment de préférence ≥ 7 mm.

9. Procédé pour isoler une lenticule (15) dans la cornée (12) d'un œil (11),
- au moins une surface de coupe (16, 17) étant spécifiée dans la cornée (12), laquelle délimite la lenticule (15) et suit un tracé n'ayant pas un écart constant par rapport à la surface avant (18) de la cornée (12),
- une trajectoire (19) qui se trouve dans la surface de coupe (16, 17) étant spécifiée,
- un rayonnement laser pulsé (6) étant délivré dans la cornée (12), avec
- une fréquence d'impulsions de 1,2 MHz à 10 MHz,
- une énergie d'impulsion de 1 nJ à moins de 100 nJ et
- une longueur d'onde qui pénètre dans la cornée (12),
- une optique de rayonnement (8) étant utilisée, laquelle comprend un champ d'image et focalise le rayonnement laser pulsé (6) dans la cornée (12) en un foyer (13) qui se trouve à l'intérieur du champ d'image et présente un diamètre maximal inférieur à 3 µm, et
- le foyer (13) étant décalé dans la cornée (12) et à l'intérieur du champ d'image, le foyer (13) migrant le long d'une trajectoire (19) lorsque le champ d'image est au repos,
- le procédé n'exécutant pas un traitement chirurgical de l'œil humain ou animal vivant.

10. Procédé selon la revendication 9, l'énergie d'impulsion ayant une valeur égale ou supérieure à 10 nJ et/ou l'énergie d'impulsion ayant une valeur de 10 nJ à 80 nJ.

11. Procédé selon l'une des revendications 9 ou 10, la longueur d'onde ayant une valeur de 1030 nm à 1060 nm, la longueur d'impulsion étant, en option, inférieure à 1 ps.

12. Procédé selon l'une des revendications 9 à 11, la longueur d'onde ayant une valeur de 300 nm à 400 nm, la fréquence d'impulsions une valeur non supérieure à 2 MHz et le foyer un diamètre maximal inférieur ou égal à 2 µm.

13. Procédé selon l'une des revendications 9 à 12, un verre de contact (10) étant posé sur l'œil, lequel comprenant une surface de contact posée sur la surface avant (18) de la cornée (12) ayant un rayon de courbure non supérieur à 50 mm, de préférence non supérieur à 20 mm.

14. Procédé selon l'une des revendications 9 à 13, l'optique de rayonnement (8) ayant une ouverture numérique d'au moins 0,33.

15. Procédé selon l'une des revendications 9 à 14, l'optique de rayonnement (8) possédant dans la cornée (12) de l'œil (11) un champ optique ayant un diamètre ≥ 3 mm, notamment ≥ 6 mm et notamment de préférence ≥ 7 mm.
